(19) Europäisches Patentamt
European Patent Office
Office européen des brevets

(11) EP 0 395 122 B1

(12) EUROPÄISCHE PATENTSCHRIFT

(45) Veröffentlichungstag und Bekanntmachung des Hinweises auf die Patenterteilung:
**19.06.1996 Patentblatt 1996/25**

(21) Anmeldenummer: **90111597.2**

(22) Anmeldetag: **10.02.1987**

(51) Int Cl.$^{6}$: **C09D 5/44**, C09D 175/12, C08G 71/04, C08G 18/64, C08G 18/80, C08G 59/02, C08G 59/40

# (54) Elektrotauchlacke auf Basis von Aminourethanen

Electrodeposition paints with a base of aminourethanes

Peintures électrophorétiques à base d'amino-uréthanes

(84) Benannte Vertragsstaaten:
**AT BE CH DE ES FR GB GR IT LI LU NL SE**

(30) Priorität: **13.02.1986 DE 3604434**
**19.07.1986 DE 3624454**

(43) Veröffentlichungstag der Anmeldung:
**31.10.1990 Patentblatt 1990/44**

(62) Anmeldenummer der früheren Anmeldung nach Art. 76 EPÜ: **87101797.6**

(73) Patentinhaber: **HOECHST AKTIENGESELLSCHAFT**
**D-65926 Frankfurt am Main (DE)**

(72) Erfinder:
- **Walz, Gerd, Dr.**
  **D-6200 Wiesbaden (DE)**
- **Hönel, Michael, Dr.**
  **D-6200 Wiesbaden (DE)**
- **Brindöpke, Gerhard, Dr.**
  **D-6231 Sulzbach (DE)**
- **Sprenger, Walter**
  **D-6110 Dieburg (DE)**
- **Finke, Manfred, Dr.**
  **D-6233 Kelkheim (Taunus) (DE)**
- **Lenz, Rüdiger, Dr.**
  **D-6000 Frankfurt am Main (DE)**

(56) Entgegenhaltungen:
**EP-A- 0 121 837** **US-A- 2 802 022**
**US-A- 3 084 140** **US-A- 4 484 994**

EP 0 395 122 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung bezieht sich auf härtbare, wasserdispergierbare Bindemittel auf Basis von Umsetzungsprodukten von modifizierten 2-Oxo-1,3-dioxolanen und primären Aminen, die mindestens eine primäre Aminogruppe und zusätzlich weitere basische Gruppierungen enthalten, und die insbesondere aus wäßriger Phase bei der kationischen Elektrotauchlackierung abgeschieden werden können.

In der DE-PS 22 52 536 werden selbst vernetzende Bindemittel für die kathodische Elektrotauchlackierung beschrieben, die aus einem Polyurethanharz, das durch Umsetzung von Epoxyd-Amin-Addukten, hergestellt aus einer epoxygruppenhaltigen organischen Verbindung mit einem sekundären Amin und einem teilweise blockierten Polyisocyanat, erhalten worden sind. Als Verkappungsmittel werden vorzugsweise primäre aliphatische Alkohole eingesetzt. Die Bindemittel müssen jedoch im allgemeinen bei relativ hohen Einbrenntemperaturen z.B. 180 °C gehärtet werden.

Kathodische Elektrotauchlacke werden auch in der EP-A 0 121 837 und in den korrespondierenden deutschen Offenlegungsschriften 33 11 517 und 33 11 518 beschrieben, bei denen Polyamine mit mindestens zwei primären Aminogruppen mit Epoxydharzen zu Epoxid-Amin-Addukten umgesetzt und die erhaltenen Reaktionsprodukte mit Alkylencarbonat unter Bildung von β-Hydroxyurethanen zur Reaktion gebracht werden. Die erhaltenen β-Hydroxyurethane benötigen im allgemeinen zwar niedrigere Härtungstemperaturen als die vorstehend beschriebenen Bindemittel, jedoch ist die Einführung der primären Aminogruppen umständlich und benötigt zusätzliche Verfahrensschritte.

In der US-A 3 084 140 wird die Synthese von Polyhydroxy-Polyurethanen aus Biscarbonaten und Diaminen beschrieben.

In der US-A 2 802 022 wird ein Verfahren zur Herstellung von Polyurethanen aus Harnstoff und primären oder sekundären β-Hydroxycarbamaten beschrieben, in denen die Hydroxygruppe direkt am aliphatischen C-Atom gebunden ist.

In der US-A 4 484 994 wird die Herstellung eines kathodisch abscheidbaren Polymeren beschrieben, das mindestens eine tertiäre Aminogruppe und mindestens zwei Hydroxyalkylcarbamat-Gruppen pro Molekül aufweist. Bei der Synthese werden ausschließlich niedermolekulare Bis-Carbonate eingesetzt.

In der EP-Offenlegungsschrift 0 119 769 sind verschiedene Möglichkeiten angeführt, tertiäre Aminogruppen in das Grundgerüst eines Harzes einzuführen. Neben der Umsetzung von Epoxyverbindungen mit Aminkomponenten nach verschiedenen Verfahren werden auch solche von α,β-ungesättigte Carboxylgruppen enthaltenden Harzen oder Carboxylgruppen enthaltenden Harzen mit Aminokomponenten beschrieben. Die erhaltenen Verbindungen werden dann mit Alkylencarbonaten zu β-Hydroxyurethangruppen enthaltenden Bindemitteln umgesetzt.

Bei der Härtung werden unter Abspaltung von Diolen, die aber physiologisch nicht unbedenklich sind Urethan- oder Harnstoffbindungen gebildet. Ein Nachteil der Umsetzung von Epoxydverbindungen mit Ketiminen ist die Einhaltung von wasserfreien Bedingungen bei der Reaktion, um eine vorzeitige Hydrolyse zu vermeiden.

Durch einen grundsätzlich anderen Weg werden die erfindungsgemäß eingesetzten Bindemittel erhalten. Hierbei werden primäre Aminogruppen und weitere basische Gruppen enthaltende Verbindungen mit Carbonaten umgesetzt, so daß stets basische Aminogruppen in den entstandenen Produkten enthalten sind. Die Härtung von Überzügen aus den erfindungsgemäßen Elektrotauchlacken kann durch Bildung von Urethan- oder Harnstoffbindungen erfolgen. Die erfindungsgemäß eingesetzten Bindemittel können durch Einbau von verkappten Isocyanatgruppen selbsthärtend sein oder können mit zugesetzten bekannten Härtern über die in den Bindemitteln vorhandenen funktionellen Gruppen gehärtet werden.

Gegenstand der Erfindung sind Elektrotauchlacke auf Wasserbasis, enthaltend Aminourethane, die erhalten werden durch Umsetzung von oligomeren oder polymeren Verbindungen, die mindestens zwei endständige 2-Oxo-1,3-dioxolan-Gruppen enthalten, mit Aminen der Formel (1)

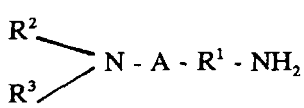

(I)

und/oder Aminen der Formel (Ia)

$$H_2N\text{-}A\text{-}R^1\text{-}NH_2 \qquad \text{(Ia)}$$

deren primäre Aminogruppe mit einem teilverkappten Polyisocyanat zu monoprimären Polyaminen umgesetzt wurde, wobei in den Formeln I und Ia

- $R^1$ ein zweiwertiger Kohlenwasserstoffrest, vorzugsweise ein geradkettiger oder verzweigter Alkylenrest mit 2 bis 18 C-Atomen,
- $R^2$ Wasserstoff, Alkyl mit 1 bis 8 C-Atomen oder Hydroxyalkyl mit 1 bis 8 C-Atomen im Alkylrest,
- $R^3$ Alkyl mit 1 bis 8 C-Atomen oder Hydroxyalkyl mit 1 bis 8 C-Atomen im Alkylrest, wobei $R^2$ und $R^3$ auch eine

cyclische Ringverbindung bilden können und

A eine chemische Bindung oder $-(R^1-NH)_r-NH-$ ist, worin r Null oder eine ganze Zahl von 1 bis 6 ist und $R^1$ die obige Bedeutung hat, und wobei zumindest ein Teil der basischen Aminogruppen dieser Aminourethane neutralisiert ist.

Die Stoffmenge an Aminen der Formeln I und/oder Ia beträgt im allgemeinen 35 bis 85 mol, vorzugsweise 50 bis 60 mol, und die der oligomeren oder polymeren Verbindungen mit mindestens zwei endständigen 2-Oxo-1,3-dioxolangruppen 15 bis 65 mol, vorzugsweise 40 bis 60 mol pro 100 mol der Reaktanden.

Vorzugsweise besitzen die erfindungsgemäß eingesetzten Aminourethane die allgemeine Formel (II)

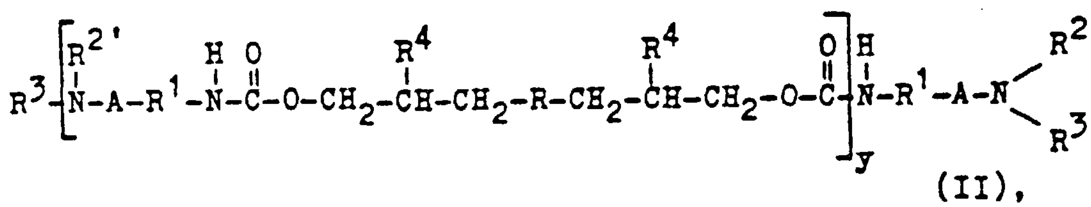

(II),

in der $R^1$ bis $R^3$ sowie A die obige Bedeutung haben

$R^4$ Hydroxyl oder den Rest

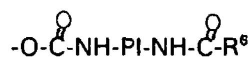

darstellt, in dem PI der Rest eines Polyisocyanats nach Entfernen von zwei -NCO-Gruppen und $R^6$ der Rest eines aliphatischen, cycloaliphatischen oder alkylaromatischen einwertigen Alkohols, eines Aminoalkohols, eines Ketoxims, einer CH- oder NH-aciden Verbindung ist,

$R^{2'}$ gleich $R^2$ ist, mit der Maßgabe, daß $R^{2'}$ nur Wasserstoff darstellt, falls das betreffende Stickstoffatom nicht am Kettenende steht,

y eine ganze Zahl von 1 bis 10, vorzugsweise 1 bis 5 bedeutet und

R gleich und verschieden sein kann und für den Rest eines Diglycidylethers oder -esters, der gegebenenfalls auch $(NR_2)$-Gruppen enthalten kann, wobei $R^2$ die obige Bedeutung hat, oder für einen zweiwertigen Kohlenwasserstoffrest, vorzugsweise einen gegebenenfalls substituierten, verzweigten oder unverzweigten Alkylenrest mit 2 bis 18 C-Atomen, vorzugsweise 2 bis 10 C-Atomen, steht.

Insbesondere ist y in der vorstehenden Formel (II) gleich 1, d.h. die besonders bevorzugten Aminourethane besitzen erfindungsgemäß die allgemeine Formel (IIa)

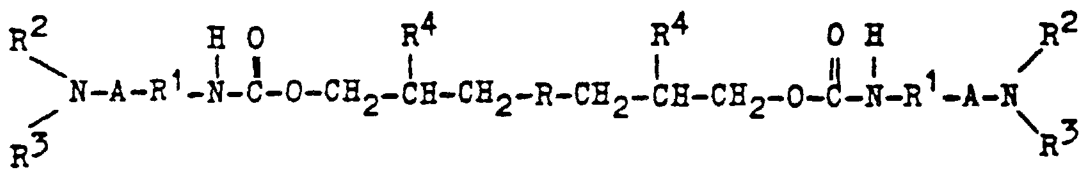

(IIa),

in der $R^1$ bis $R^4$ sowie A die obige Bedeutung haben.

Was den Rest R in diesen Formeln (II) und (IIa) anbelangt, so kann dieser nach einer Ausführungsform der Erfindung die nachstehende Formel aufweisen

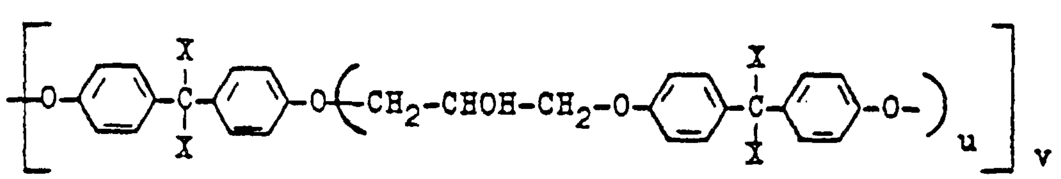

,

in der X Wasserstoff oder Methyl, u 0 bis 5 und v 1 bis 20, vorzugsweise 1 bis 6 ist. Die Werte für u und v sind als statistisches Mittel anzusehen, da durch die Molekulargewichtsverteilung der Glycidylether ein großer Bereich einbezogen werden kann.

Eine weitere Ausführungsform betrifft solche Aminourethane, bei denen R in den Formeln (II) und (IIa) den Rest

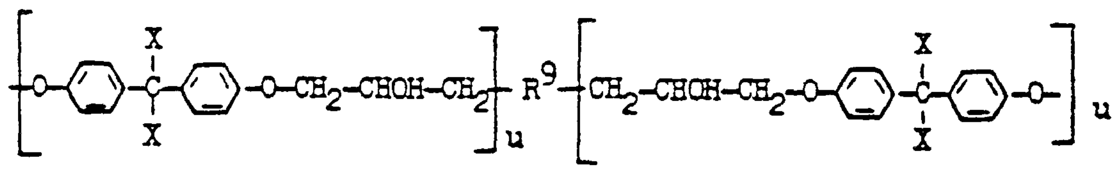

darstellt, worin X und u die genannte Bedeutung haben und $R^9$ 0-Alkyl-O, N-Alkyl-N mit jeweils 2 bis 18 C-Atomen im Alkylrest sowie der Rest von Polyaminen, Polyolen, Polycaprolactonpolyolen, OH-gruppenhaltigen Polyestern, Polyethern. hydroxy-, carboxyl- und aminofunktionellen Polymerölen, Polycarbonsäuren, hydroxy- oder aminofunktionellen Polytetrahydrofuranen und Reaktionsprodukten von Polyaminen mit Glycidylestern von in α-Stellung verzweigten Carbonsäuren mit 8 bis 14 C-Atomen (sog. $^{(R)}$Versaticsäuren) ist.

Vorzugsweise stellt $R^9$ ein Rest gemäß einer der folgenden Formeln dar:

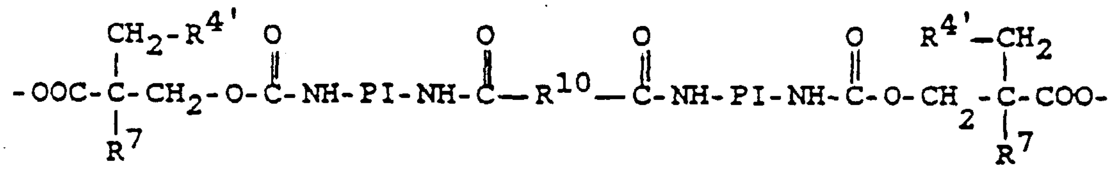

wobei die Reste $R^7$ und PI die obige Bedeutung haben, $R^4$ neben $R^4$ auch zusätzlich Wasserstoff sein kann, $R^{10}$ für die unter $R^9$ angeführten Reste mit Ausnahme der Polycarbonsäuren und der carboxylfunktionellen Polymeröle steht oder den Rest

$$-OOC-R^{11}-CO-R^{10}-CO-R^{11}-COO-$$

bedeutet, in dem $R^{10}$ wie vorstehend definiert ist und $R^{11}$ den aliphatischen, cycloaliphatischen oder aromatischen Rest einer Polycarbonsäure darstellt. Beispiele hierfür sind o- und p-Phthalsäure, Tetrahydrophthalsäure, Bernsteinsäure, Trimellithsäure, wobei letztere vorzugsweise mit einem primären Diamin als Rest $R^{10}$ unter Imidbildung umgesetzt worden ist.

Nach einer anderen Ausführungsform besitzt R in Formel (II) die Struktur

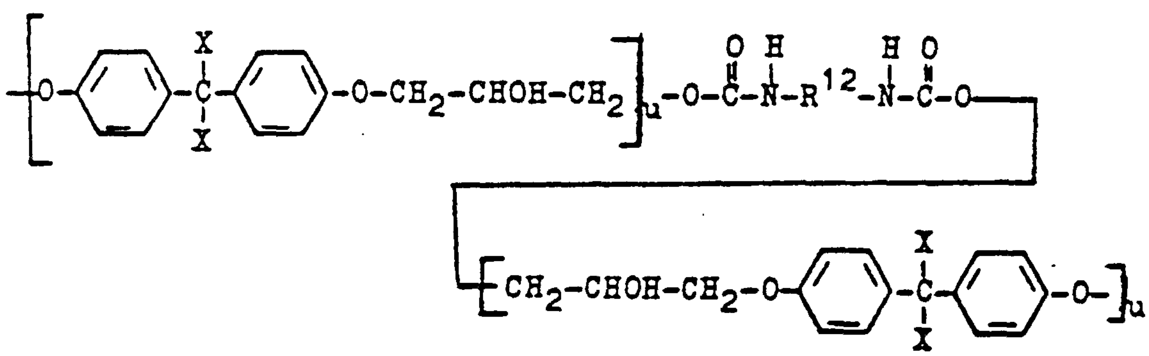

worin X und u die genannte Bedeutung haben und $R^{12}$ Alkylen mit 2 bis 18 C-Atomen, der Rest eines Poly-(sek.)Amins oder aminofunktionelle Polytetrahydrofuran ist.

Nach einer weiteren Ausführungsform besitzt R in Formel (II) die Struktur

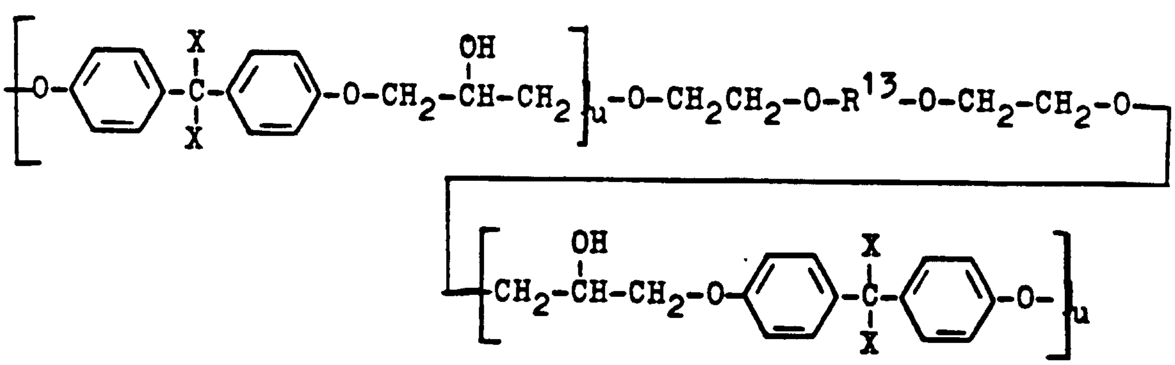

worin X und u die genannte Bedeutung haben, u vorzugsweise aber 1 ist, und $R^{13}$ den Rest

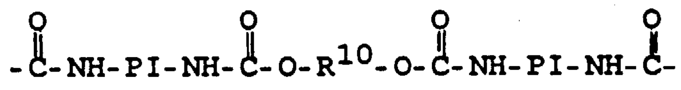

oder

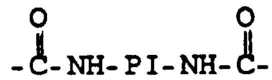

darstellt, wobei $R^{10}$ und PI die schon genannten Definitionen haben.

Weiterhin können ein Teil oder alle der vorhandenen Hydroxyl- oder Aminogruppen in den Verbindungen der Formeln (II) oder (IIa) mit teilverkappten Polyisocyanaten umgesetzt sein.

Von besonderem Interesse sind solche Produkte, bei denen als weitere Aminogruppe eine primäre oder sekundäre Aminogruppe vorhanden ist. Auf diese Weise kann der nach der Härtung verbleibende Anteil von basisch wirkenden Stickstoffgruppierungen im Molekül deutlich vermindert werden.

Die Herstellung der erfindungsgemäß als Bindemittel eingesetzten Aminourethane kann in der Weise erfolgen, daß man (α) Polyamine der allgemeinen Formel (I)

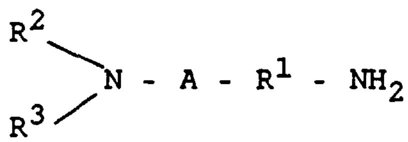 (I)

in der $R^1$ bis $R^3$ sowie A die obige Bedeutung besitzen, mit (β) oligomeren oder polymeren Verbindungen, die mindestens eine, vorzugsweise zwei bis fünf und insbesondere zwei endständige 2-Oxo-1,3-dioxolan-Gruppen enthalten, gegebenenfalls in Gegenwart von Kettenstoppern umsetzt.

Die Menge an Polyamin (α) beträgt im allgemeinen 35 bis 85 Mol-%, vorzugsweise 50 bis 60 Mol-% und die von Komponente (β) 65 bis 15 Mol-%, vorzugsweise 40 bis 50 Mol-%.

Falls in der obigen Formel (I) der Komponente (α) das Symbol A für eine chemische Bindung steht, besitzen die erfindungsgemäß einsetzbaren Polyamine die Formel

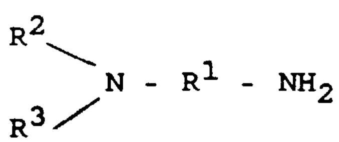 (Ia)

mit den für $R^1$ bis $R^3$ oben angegebenen Definitionen. Beispiele für derartige Polyamine sind:
N-Methylethylendiamin, Hydroxyethylaminoethylamin,
-propylamin, N,N′-dimethylethylendiamin,
N,N′-Dimethylpropylendiamin,
N,N′-Dihydroxyethylethylendiamin;
Ethylendiamin, Propylendiamin, Hexamethylendiamin,
Octamethylendiamin, Triacetondiamin, Dioxadecandiamin,
Dioxadodecandiamin und höhere Homologe; cycloaliphatische Diamine wie 1,4-Cyclohexandiamin, 4,4′-Methylen-bis-cyclohexylamin und 4,4′-Iso-propylenbis-cyclohexylamin, Isophorondiamin und N-Aminoethylpiperazin. Selbstverständlich können auch Gemische dieser Polyamine untereinander, beispielsweise auch di-primäre mit mono-primären, wie auch mit denen gemäß der nachstehenden Formel (Ib) verwendet werden. Die dabei resultierenden Mischungen von Aminourethanen sind für die erfindungsgemäßen Zwecke gleichfalls gut geeignet.

In der obigen Formel (I) kann A auch für $-(R^1NH)_r-R^1$ NH- stehen. Die Komponente (α) weist dann also die Formel

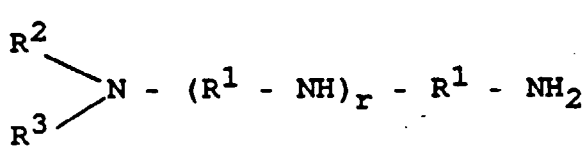 (Ib)

auf, in der $R^1$ bis $R^3$ die obige Bedeutung haben und r für 1 bis 6, vorzugsweise 1 bis 4 steht.

Beispiele hierfür sind: Diethylentriamin, Dipropylentriamin, Bishexamethylentriamin, Triethylentetramin, Tetraethylenpentamin, Pentaethylenhexamin, Heptaethylenoctamin und ähnliche.

Die oligomere oder polymere Verbindung (β) besitzt vorzugsweise die allgemeine Formel (III)

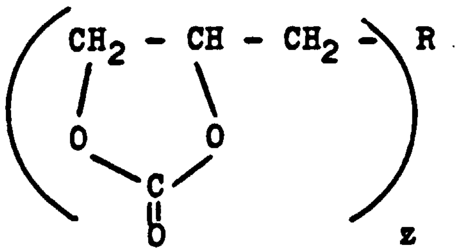

(III),

in der R die obige Bedeutung hat, jedoch in seiner Wertigkeit z entspricht, und z für ganze Zahlen von 1 bis 5, vorzugsweise 2 und 3 und insbesondere 2 steht.

In dieser Formel ist R bevorzugt der Rest eines Polyethers, Polyetherpolyols, Polyesters, Polyesterpolyols, ferner ein Alkylenrest, ein Poly(sek.)aminrest oder auch ein Umsetzungsprodukt einer Epoxy-Carbonat-Verbindung mit Polyaminen, aliphatischen oder aromatischen Polyolen von Novolaken, Polycaprolactonpolyolen, OH-gruppenhaltigen Polyestern, Polyethern, Polyglykolen, hydroxy-, carboxyl- und aminofunktionellen Polymerölen, Polycarbonsäuren, hydroxy- oder aminofunktionellen Polytetrahydrofuranen und Reaktionsprodukten von Polyaminen mit Glycidylestern von α-Alkylalkanmonocarbonsäuren und/oder α,α-Dialkylalkanmonocarbonsäuren der Summenformel $C_{12-14}H_{22-26}O_3$ (Versaticsäuren), einzeln oder im Gemisch. Die α-Alkylalkansäure- und α,α-Dialkylalkansäure-Gemische stellen Monocarbonsäuren dar, die eine $C_9$-, $C_{10}$- und $C_{11}$-Kette enthalten. Die Ester werden nachfolgend als Versaticsäureglycidylester bezeichnet. Hydroxyl-, carboxyl- und aminofunktionelle Polymeröle sind handelsüblich und stellen modifizierte Polybutadiene mit einem Molekulargewicht von 800 bis 10000 dar.

Das Molekulargewicht $\overline{M}_w$ (Gewichtsmittel, bestimmt mit der Gelchromatographie, Polystyrolstandard) der Verbindungen gemäß Formel (III) liegt im allgemeinen zwischen 300 und 10000, vorzugsweise zwischen 800 und 4000.

Die Verbindungen gemäß Formel (III), wie auch die nachfolgend beschriebenen Verbindungen (IV) und (V), können durch Anlagerung von $CO_2$ an die entsprechenden epoxidgruppenhaltigen Verbindungen hergestellt werden. Derartige Verfahren sind bespielsweise in der PCT(WO)-Patentanmeldung 84/03 701 sowie in den DE-Patentanmeldungen P 35 29 263.6 und P 36 00 602.5 beschrieben. Auf deren Offenbarung, einschließlich der bevorzugten Ausführungsformen, wird hier Bezug genommen. Geeignete Ausgangspolyepoxide sind beispielsweise in Wagner/Sarx, "Lackkunstharze", Carl Hanse Verlag (1971), S. 174 ff sowie in der EP-Offenlegungsschrift 60 506 aufgeführt, auf die hier gleichfalls Bezug genommen werden.

Bevorzugte Ausgangsstoffe zur Herstellung der cyclischen Carbonate (III) und der gemischten Epoxyd-Carbonat-Verbindungen (IV) sind die Polyglycidylether von Polyphenolen z.B. Bisphenol A. Die Glycidyläther erhält man beispielsweise durch Umsetzung eines Polyphenols mit Epichlorhydrin. Polyphenole sind beispielsweise Bis-(4-hydroxyphenyl)-2,2-propan, Bis(4-hydroxyphenyl)-methan, 4,4′-Dihydroxybenzophenon, Bis(4-hydroxyphenyl)-1,1′-ether, Bis (4-hydroxyphenyl)-1,1′isobutan, Bis(2-hydroxynaphthyl)-methan, 1,5-Dihydroxynaphthalin. Vorzugsweise sind zusätzlich freie Hydroxylgruppen zu den Epoxydgruppen im Polyglycidylether des Polyphenols enthalten.

Die Umsetzung der Komponenten (α) und (β) erfolgt im allgemeinen in den erforderlichen stöchiometrischen Verhältnissen bzw. Mengen nach üblichen Methoden bei erhöhten Temperaturen, gegebenenfalls unter Zusatz von Katalysatoren und gegebenenfalls unter Zusatz von inerten Lösungsmitteln. Die Umsetzung in Gegenwart von gegenüber der Cyclocarbonatgruppe inerten Lösungsmitteln stellt dabei eine bevorzugte Verfahrensvariante dar. Bei der stöchiometrischen Bewertung der Ausgangsprodukte und auch der Umsetzungsprodukte bezüglich des Fortgangs der Umsetzung werden bei den Verbindungen der Komponente (α), die Aminzahl, deren Ermittlung in üblicher Weise durch Perchlorsäuretitration erfolgen kann, und bei den Verbindungen der Komponente (β) die Cyclocarbonat-Äquivalenzzahl, die in üblicher Weise durch Titration mit Kaliumhydroxidlösung bestimmt werden kann, zugrunde gelegt. Bei der Umsetzung der Komponenten (α) und (β) können die Polyaminoverbindungen einzeln oder als Gemische oder auch zeitlich nacheinander der Reaktion zugeführt werden, gegebenenfalls gelöst in inerten organischen Lösungsmitteln. In analoger Weise können auch einzelne oder verschiedene modifizierte cyclische Carbonate der Komponente (β) einzeln oder als Gemische oder auch zeitlich nacheinander, vorzugsweise im Gemisch mit gegenüber Cyclocarbonatgruppen inerten organischen Lösungsmitteln, der Reaktion zugeführt werden.

Es ist bei der Umsetzung zu beachten, daß solche Reaktions - und Verfahrensbedingungen eingehalten werden, unter denen die Cyclocarbonatgruppen der Komponente (β) nur mit den primären Aminogruppen der Komponente (α) reagieren können, was nach bekannten Methoden erreichbar ist, ohne daß auch entsprechende Reaktionen mit den vorhandenen sekundären Aminogruppen stattfinden, die reaktionsträger sind.

Gemäß einer Ausführung der Erfindung ist es möglich, das Umsetzungsprodukt als selbsthärtende Verbindung herzustellen. Dabei werden an die gegebenenfalls im cyclischen Carbonat vorhandenen Hydroxyl- bzw. sekundären Aminogruppen teilverkappte Polyisocyanate angelagert. Die Hydroxylgruppen können auch durch Anlagerung von Formaldehyd an einen aromatischen Kern der phenolischen Komponente gebildet werden. Diese Reaktion wird unter

solchen Bedingungen durchgeführt, daß das cyclische Carbonat nicht angegriffen wird.

Polyisocyanate, die für diese Verbindungen mit dem Rest PI eingesetzt werden, können beliebige Polyisocyanate sein, beispielsweise ein aliphatisches, cycloaliphatisches oder aromatisches Polyisocyanat. Ein Teil der Isocyanatgruppen kann in bekannter Weise mit üblichen Verkappungsmitteln umgesetzt worden sein. Typische Beispiele für die verwendeten Polyisocyanate sind Xylylendiisocyanat, Diphenylmethan-4,4′-diisocyanat, Triphenylmethyl-4,4′-triisocyanat, Triphenylmethantriisocyanat, Polyphenyl-polymethyl-isocyanat, 2,2,4(2,4,4)-Methylcyclohexyldiisocyanat, Dicyclohexylmethyldiisocyanat, Diethylfumarhexylisocyanat, Bis-(3-Methyl-4-isocyanatocyclo-hexyl-)methan, 2,2-Bis-(4-isocyanatocyclohexyl-)propan, den Methylester des Lysindiisocyanats, das Biuret des Hexamethylendiisocyanats, Diisocyanate dimerer Säuren, 1-Methyl-benzol-2,4,5-triisocyanat, Biphenyl-2,4,4′-triisocyanat, das Triisocyanat aus 3 Mol Hexamethylendiisocyanat und 1 Mol Wasser mit 16 % NCO-Gehalt und weitere, wenigstens zwei NCO-Gruppen pro Molekül enthaltende Verbindungen, vorzugsweise Isophorondiisocyanat, Hexamethylendiisocyanat sowie Tri- und Tetramethylhexamethylendiisocyanat, insbesondere aber 2,4- oder 2,6-Toluylendiisocyanat oder Gemische dieser Verbindungen. Die in den Verbindungen dem Rest PI zugrundeliegenden Polyisocyanate können gleich oder verschieden sein.

Neben diesen einfachen Polyisocyanaten sind auch solche geeignet, die Heteroatome in dem die Isocyanatgruppen verknüpfenden Rest enthalten. Beispiele hierfür sind Polyisocyanate, die Carbodiimidgruppen, Allophonatgruppen, Isocyanuratgruppen, Urethangruppen, acylierte Harnstoffgruppen und Biuretgruppen aufweisen.

Geeignete Polyisocyanate sind schließlich auch die bekannten, endständigen Isocyanatgruppen aufweisenden Präpolymere, wie sie insbesondere durch Umsetzung der obengenannten einfachen Polyisocyanate, vor allem Diisocyanate mit unterschüssigen Mengen an organischen Verbindungen mit mindestens zwei gegenüber Isocyanatgruppen reaktionsfähigen Gruppen zugänglich sind. Bevorzugt werden diese Präpolymeren allerdings als externe Härterkomponente bei den fremdvernetzenden Systemen eingesetzt.

Als Verkappungsmittel eignen sich aliphatische, cycloaliphatische oder alkylaromatische (einwertige) Alkohole z. B. niedere aliphatische Alkohole wie Methyl-, Äthyl-, die verschiedenen Propyl-, Butyl- und Hexylalkohole, Heptyl-, Octyl-, Nonyl-, Decylalkohol und ähnliche, ferner ungesättigte Alkohole wie Allylalkohole, cycloaliphatische Alkohole wie Cyclopentanol, Cyclohexanol, alkylaromatische Alkohole wie Benzylalkohol, Methyl sowie p-Methoxy und p-Nitrobenzylalkohol und Monoäther von Glykolen wie Ethylen-glykolmonoethyläther, -monobutyläther und ähnliche. Weitere Verkappungsmittel sind Ketoxime, zweckmäßigerweise mit 3 bis 20 C-Atomen, vorzugsweise 3 bis 10 C-Atomen, wie Acetonoxim, Methylethylketonoxim (=Butanonoxim), Hexanonoxim (wie Methyl-Butylketonoxim), Heptanonoxim (wie Methyl-n-Amylketonoxim), Octanonoxim und Cyclohexanonoxim, CH-acide Verbindungen wie Malonsäurealkylester, Acetessigester sowie Cyanessigester mit jeweils 1 bis 4 C-Atomen in der Estergruppe, NH-acide Verbindungen wie Caprolactam, Aminoalkohole wie Diäthyläthanolamin. Das als Blockierungsmittel bekannte Phenol kann in solchen Fällen eingesetzt werden, bei denen das Umsetzungsprodukt zur Herstellung von lösungsmittelhaltigen Lacken verwendet wird.

Die erfindungsgemäß eingesetzten Aminourethane können nach verschiedenen Verfahren hergestellt werden. Beispielsweise kann eine Verbindung der Formel (III), in der R z.B. der Rest eines Polyglycidyläthers vom Bisphenol A sein kann, bei dem die Epoxydgruppen zu Carbonatgruppen umgewandelt worden sind, mit einer Verbindung der Formel (I), in der $R^2$ und $R^3$ die angegebene Bedeutung haben, umgesetzt werden. Will man ein selbsthärtendes Produkt erhalten, wird die Verbindung (III) zuerst über vorhandene OH-Gruppen mit einem teilverkappten Polyisocyanat unter Urethanbildung umgesetzt, worauf die Reaktion mit der Verbindung (I) erfolgt. Ferner kann man teilverkappte Polyisocyanate zuerst mit einem Aminoalkylierungsprodukt, das durchschnittlich mindestens eine NH-Gruppe pro Molekül aufweist und das aus Phenol und/oder einem substituierten Phenol, vorzugsweise Monoalkyl- oder Monoaryl- oder Monoaralkylphenol mit einer oder gegebenenfalls zwei phenolischen Hydroxylgruppen, einem primären Alkylamin und/oder primären Alkanolamin und/oder primär-tertiären Alkyldiamin und Formaldehyd oder einer formaldehydabspaltenden Verbindung erhalten worden ist, in einer weiteren Reaktionsstufe mit einem gemischten Epoxid-Carbonat (s. nachfolgende Formel IV) d.h. einer Verbindung, in der neben cyclischen Carbonatgruppen noch Ausgangs-Epoxyidgruppen vorhanden sind, umsetzen. So erhält man auch wieder Verbindungen mit zwei Carbonatgruppen der Formel (III).

Andererseits ist es aber auch möglich, den erfindungsgemäß eingesetzten Aminourethanen beispielsweise einen üblichen Härter zuzusetzen, wie sie für fremdvernetzende 2-Komponentenlacke verwendet werden. Hierfür kommen beispielsweise in Frage verkappte Polyisocyanate, etwa wie sie oben für die selbsthärtenden Aminourethane beschrieben sind, weiter β-Hydroxyester von mindestens bifunktionellen Polycarbonsäuren, Umsetzungsprodukte von Malonsäuredialkylestern mit Aldehyden und Ketonen, die unter Wasserabspaltung zu ungesättigten Dicarbonsäureestern reagieren (Knoevenagel'sche Synthese), Umesterungshärter sowie Michael-Additionsprodukte, beispielsweise wie sie in den DE-Offenlegungsschriften 33 15 469 und 34 17 441 sowie in der DE-Patentanmeldung P 36 02 981.5 beschrieben sind. Auf diese Literaturstellen, einschließlich der bevorzugten Ausführungsformen, wird hiermit Bezug genommen.

Als Härterkomponente für die erfindungsgemäß verwendeten Aminourethane eignen sich auch epoxidgruppenhaltige Verbindungen, wie z. B. niedermolekulare Polyepoxide, epoxidgruppenhaltige Copolymerisate sowie Di- oder Polyglycidylether von aliphatischen oder aromatischen Alkoholen. Weiterhin sind hier als Härterkomponenten auch oligomere oder polymere Verbindungen zu nennen, welche mindestens zwei 1,3-Dioxolan-2-on-Gruppen oder mindestens eine 1,3-Dioxolan-2-on-Gruppe und eine Epoxidgruppe pro Molekül enthalten: hierzu gehören beispielsweise die Verbindungen (III) und (IV).

Um der unter Verwendung dieser Umsetzungsprodukte erhaltenen Lackschicht die nötige Flexibilisierung zu geben, können die Verbindungen (I) und (III) bereits die nötigen Voraussetzungen mitbringen. Andererseits ist es auch möglich, entweder über den eingebauten Härter in Form von teilverkappten Polyisocyanaten oder über den zugemischten Härter die benötigte Flexibilisierung in das System einzubringen.

Diese Flexibilisierung kann aber auch, wie vorstehend bereits erwähnt, über Verbindungen gemäß der Formel (III) erfolgen. Hierbei geht man beispielsweise von gemischten Epoxyd-Carbonaten aus, die solche der allgemeinen Formel (IV) darstellen

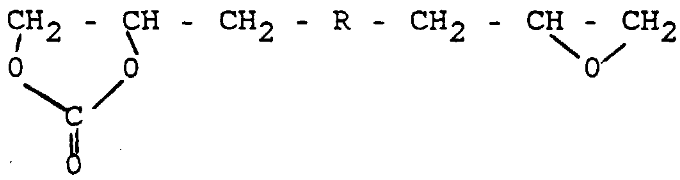

(IV)

wobei R′ die in Formel (III) genannte Bedeutung hat. Diese gemischten Epoxid-Carbonate werden mit Verbindungen umgesetzt, die einen flexibilisierenden Effekt auf das Molekül ausüben, beispielsweise die bereits früher genannten Polyamine, aliphatische oder aromatische Polyole, wie Diole, Triole oder Tetraole, beispielsweise Ethylen- oder Propylenglykol, Polyalkylenglykole, Neopentylglykol, Glycerin, Trimethylolpropan, Pentaerythrit sowie Polycaprolactonpolyole, weiter OH-gruppenhaltige Polyester, Polyether, Polyglykole, hydroxy-, carboxyl- und aminofunktionelle Polymeröle, Polycarbonsäuren, hydroxy- und aminofunktionelle Polytetrahydrofurane und Umsetzungsprodukte von Polyaminen mit Versaticsäureglycidylestern. Diese Umsetzungen werden unter Bedingungen vorgenommen, bei denen nur die verbliebenen Epoxydgruppen reagieren und die Carbonatgruppen nicht angegriffen werden. Auf diese Weise erhält man auch wieder Verbindungen der Formel (III), die endständige cyclische Carbonatgruppen tragen, die mit den Aminoverbindungen (I) umgesetzt werden können.

Die endständigen Aminogruppen können mit sogenannten Kettenstoppern, das sind z.B. teilverkappte Polyisocyanate umgesetzt werden, wobei die Umsetzungen gleichzeitig oder in mehreren getrennten Stufen durchgeführt werden können. Hierbei ist es auch möglich, zunächst die Polyamine (I), in denen zumindestens einer der beiden Reste $R^2$ und $R^3$ Wasserstoff bedeutet, in entsprechender Weise mit diesen Kettenstoppern (Blockierungsmitteln) umzusetzen und diese so partiell blockierten, nur noch mono-primären Polyamine, gegebenenfalls zusammen mit diprimären Polyaminen, mit den Verbindungen (β) zur Reaktion bringen.

Im Falle von teilverkappten Polyisocyanaten reagieren diese Verbindungen zuerst mit einer freien $NH_2$-Gruppe. Prinzipiell kann jede Aminreaktion herangezogen werden, die bevorzugt an der primären Aminogruppe ansetzt, bevor die im Molekül enthaltenen sekundären Aminogruppen reagieren. Andererseits kann die zum Kettenabbruch eingesetzte Verbindung auch zur Flexibilisierung des resultierenden Lackes dienen, indem entsprechende langkettige Verbindungen, die in der Praxis bekannt sind, eingebaut werden.

Die Temperatur bei der Umsetzung der Komponenten (α) und (β) beträgt im allgemeinen 20 bis 150, vorzugsweise 50 bis 100°C. Die Umsetzung wird im allgemeinen so lange vorgenommen bis z.B. eine konstante Aminzahl der Aminourethane erreicht ist.

Die Bedingungen bei der Herstellung der Ausgangskomponenten (siehe auch Abschnitt I bis V der Beispiele) sowie bei der Herstellung der erfindungsgemäß eingesetzten Aminourethane (→ Abschnitt VI der Beispiele), können in weiten Grenzen schwanken. Die hierzu in den Beispielen gemachten Angaben sollen daher nur richtungsweisend sein. Der Einsatz von geringen Mengen eines geeigneten Katalysators, wie basische Verbindungen, ist bei den Reaktionen auch möglich.

In den erfindungsgemäßen Elektrotauchlacken, die aus wäßriger Lösung bei einem Bad-pH zwischen etwa 3 und 9 elektrisch abgeschieden werden können, sind die basischen Aminogruppen teilweise oder vollständig neutralisiert.

Die Neutralisation der basischen Gruppen wird im allgemeinen mit wasserlöslichen Säuren, beispielsweise Ameisensäure, Essigsäure, Milchsäure oder Phosphorsäure vorgenommen. Die Menge der Zugabe der Säure hängt im Einzelfall von den Eigenschaften des verwendeten Harzes ab und wird im allgemeinen nur so weit durchgeführt, daß das Harz solubilisiert oder dispergiert wird.

Wäßrige Zubereitungen, die einen besonders niedrigen Gehalt an flüchtigen organischen Lösungsmittel besitzen, erhält man beispielsweise durch Abdestillation der in den Bindemitteln von der Herstellung oder Lösung enthaltenen Lösungsmittel. Bevorzugt wird dieser Verfahrensschritt unter vermindertem Druck durchgeführt.

Die erfindungsgemäßen Elektrotauchlacke enthalten neben den Aminourethanen verschiedene Zusatzstoffe, wie

Pigmente, Pigmentpasten, Antioxidantien, oberflächenaktive Mittel, Lösungsmittel, Verlauf- und Verdickungsmittel, Reaktivverdünner, Katalysatoren und dergleichen zugesetzt werden. Diese Zusatzstoffe sind bekannt und werden üblicherweise in der Lackindustrie eingesetzt. Die elektrische Ablagerung der Lackteilchen erfolgt nach bekannten Verfahren, worauf hier Bezug genommen wird. Die Abscheidung kann auf allen elektrisch leitenden Substraten erfolgen, z.B. Metall wie Stahl, Kupfer, Aluminium und dergleichen.

Nach der Abscheidung wird der Überzug bei erhöhten Temperaturen, die im allgemeinen von der Beschaffenheit der Härterkomponente abhängig sind, durch übliche Verfahren gehärtet, wobei Temperaturen von 100 bis 220°, vorzugsweise 130 bis 180° verwendet werden.

In den nachfolgenden Beispielen bedeutet T Gewichtsteile und % Gewichtsprozent. Die Aminzahlen beziehen sich stets auf Festharz.

**Beispiele**

**I. Teilverkappte Polyisocyanate**

**Härtende Verbindungen**

1.) Zu 174 T Toluylendiisocyanat (2 Äquiv. NCO; 80 % 2,4-, 20 % 2,6-Isomeres) wurden bei 60 - 70°C in Gegenwart von 0,3 % Benzyltrimethylammoniumhydroxyd (R)(Triton B) als Katalysator 137 T 2-Äthylhexanol (1,05 Äquiv. OH) zugegeben und bis zu einem NCO-Wert von ca. 12.8 % umgesetzt.

**Nichthärtende Verbindungen**

2.) Zu 348 T Toluylendiisocyanat (4 Äquiv.) in 385 T Diglykoldimethyläther wurden in Gegenwart von 0,3 % Triton B als Katalysator 550 T(R) Capa 200 (2 Äquiv.; Handelsname eines Polycaprolactondiols mit einer mittleren Molmasse von 550) bei 50-70°C langsam zugegeben. Die Reaktionsmischung wurde bei dieser Temperatur bis zu einem NCO-Wert von 9,4 % geführt und hatte einen Feststoffanteil von 70 %.

**II. Umsetzungen mit teilverkappten Polyisocyanaten**

1.) Zu 540 T Capa 305 (3 Äquivalente OH; Handelsname eines Polycaprolactontriols mit einer mittleren Molmasse von 540) in 628 T Diglykoldimethyläther wurden bei 40-60°C in Gegenwart von 0,3 % Triäthylamin als basischem Katalysator 915 T der Verbindung I.1.) (3 Äquiv. NCO) zugegeben und die Reaktion fortgeführt, bis der NCO-Wert auf 0 % gesunken war. Das Produkt liegt als 70 %ige Lösung vor.

2.) Zu 268 T Dimethylolpropionsäure wurden bei 30 - 60°C in Gegenwart von 0,3 % Zinkacetylacetonat als Katalysator 615 T der Verbindung I.1) (2 Äquiv. NCO) und 1683 T der Verbindung I.2) (2 Äquiv. NCO, 70 %ig) zugegeben, gegebenenfalls nach Zugabe von 2000 T 2-Butanon als Lösungsvermittler, das nach der Umsetzung unter vermindertem Druck wieder abdestilliert werden kann. SZ = 54,5.

**III. Umsetzungen von Biscarbonaten und teilverkappten Polyisocyanaten**

1.) Zu 1759 T eines Biscarbonats auf Basis von (R)Epikote 1001 (2 Äquiv. Carbonat; 60 %ig in Diglykoldimethyläther) wurden bei 60-80°C 618 T der Verbindung I.1.) (2 Äquiv. NCO) langsam zugegeben und in Gegenwart von 0,3 % Triton B bis zu einem NCO-Wert von ca. 0 % umgesetzt. Das Produkt ist 70 %ig und hat ein Carbonatäquivalentgewicht von 835.

2.) Zu 2374 T der Verbindung IV.3 (siehe dort) (2 Äquiv. Carbonat, 2 Äquiv. sek. OH, 70 %ig) wurden bei 40-80°C in Gegenwart von 0,3 % Zinkacetylacetonat als Katalysator 307 T der Verbindung I.1) (1 Äquiv. NCO) gegeben und bis zu einem NCO-Wert von ca. 0 % umgesetzt. Mit Diglykoldimethylether wurde auf einen Festkörpergehalt von 70 % eingestellt. Carbonatäquivalentgewicht 985.

**IV. Umsetzungsprodukte Carbonat/Epoxyverbindung und Polyol**

1.) 416 T des Monocarbonats von (R)Epikote 828 (1 Äquiv. Epoxid) wurden in 310 T Ethylenglykol (5 mol) gelöst und bei 100 - 150°C unter Verwendung von 0,2 % Kaliumjodid bis zu einer Epoxidzahl von ca. 0 umgesetzt. Anschließend wurde überschüssiges Ethylenglykol unter vermindertem Druck abgezogen und der Ansatz mit Diglykoldimethylether auf 80 % Festkörpergehalt eingestellt. Carbonatäquivalentgewicht ca. 478.

2.) Beispiel IV.1.) wurde wiederholt mit dem Unterschied, daß die Verbindung IV.1) durch die in der Tabelle 2 angeführte Hydroxylverbindung unter Verwendung von 0,3 % Bortrifluorid-Diethyletherat ersetzt wurde. Die dabei erhaltene Verbindung besaß das in der Tabelle 2 wiedergegebenen Carbonatäquivalentgewicht.

Tabelle 2

| | Ersatz v. IV.1) durch T (2 equiv. OH, 70%ig) | Carbonatäquivalentgewicht |
|---|---|---|
| | V.2.) Capa 200 | |
| IV. Beisp. 2 | 786 | 691 |

3.) 832 T des Monocarbonats von (R)Epikote 828 (2 Äquiv. Epoxid), 830 T Capa 205 und 712 T Diglykoldimethylether wurden gemischt und bei 70-140°C in Gegenwart von ca. 0,3 % Bortrifluoridetherat zur Reaktion gebracht, bis eine Epoxidzahl von ca. 0 erreicht wurde. Das 70%ige Produkt (Diglykoldimethylether) besitzt ein Carbonatäquivalentgewicht von ca. 831.

**V. Umsetzungsprodukte Carbonat/Epoxidverbindung und Carbonsäure**

1.) 3652 T der Verbindung II.2) (4 Äquiv. COOH) und 1664 T eines Monocarbonats auf Basis von (R)Epikote 828 (4 Äquiv. Epoxid) wurden in Gegenwart von 0,2 - 0,4 % (R)Cordova Accelerator AMC-2 als Katalysator bei 50 - 100°C bis zu einer Epoxidzahl von ca. 0 und einer SZ < 5 mg KOH/g FH umgesetzt. Gegebenenfalls können ca. 1000 T 2-Butanon als Lösungsvermittler zugesetzt werden, die anschließend unter vermindertem Druck wieder abgezogen werden. Das Reaktionsprodukt wurde mit ca. 1000 T Diglykoldimethylether auf einen Festkörpergehalt von 70 % eingestellt. Die Reaktion kann auch in Gegenwart von Diglykoldimethylether mit 70 % Festkörperanteil unter Einsatz von 0,5 - 1 % Triethylamin bei 70-140°C bis zum Erreichen der oben angeführten Kennzahlen durchgeführt werden.

**VI. Herstellung der Bindemittel**

1.1.) Zu 1497 T eines Biscarbonats auf Basis von Epikote 1001 (2 Äquiv. Carbonat, 70 %ig in Diglykoldimethyläther) wurden 204 T N,N-Dimethylaminopropylamin bei 60-80° zugegeben und bis zum Erreichen einer Aminzahl von 89,5 mg KOH/g zur Reaktion gebracht. Das erhaltene Produkt wurde mit Methoxypropanol auf einen Festkörpergehalt von ca. 70 % eingestellt.

1.2.) 63 T der Verbindung VI.1.1.) wurden mit 37 T der Verbindung II.1.) vermischt. Nach Zugabe von 5 T Dibutylzinndilaurat und 366 T deionisiertem Wasser wurde eine 15 %ige Klarlacklösung erhalten bei einem MEQ-Wert von 70 (mmol Ameisensäure/100 g Festharz), die einen pH-Wert von 6 besaß. Die Klarlacklösung ergab bis zu 250 Volt einen abscheidbaren Film, der bei 160°C für 20 Minuten ausgehärtet wurde und eine Schichtdicke von 20 µm bei einer Abscheidespannung von 200 Volt aufwies.

2.1.) Zu x T Diamin (2 Äquivalent primär. Amin) wurden 305 T des teilverkappten Polyisocyanats I.1.) (1 Äquiv. NCO) zugegeben und bei 20-40°C gehalten, bis der NCO-Wert auf 0 % gesunken war.

Tabelle 4

| | Amin | x(T) | Aminzahl (mg KOH/g Festharz) |
|---|---|---|---|
| 2.1.1. | Diethylentriamin | 103 | 275 |
| 2.1.2. | Triethylentetramin | 146 | 373 |

2.2.) Zu 4737 T der Verbindung III.1.) (4 Äquiv. Carbonat) und 1974 T der Verbindung IV.2.) (2 Äquiv. Carbonat) wurden bei 60-80°C 352 T 4,7-Dioxadecan-1,10-diamin (4 Äquiv. prim. Amin), 408 T der obigen Verbindung 2.1.1.) (1 Äquiv. prim. Amin) und 451 T des obigen Produkts 2.1.2) (1 Äquiv. prim. Amin) zugegeben und bis zu einer Aminzahl von 28,5 mg KOH/g Festharz zur Reaktion gebracht. Anschließend wurde mit Methoxypropanol auf einen Festkörpergehalt von 70 % eingestellt. Durch Zugabe von deionisiertem Wasser ließ sich daraus eine 15 %ige Klarlacklösung bei einem MEQ-Wert von etwa 20 mit einem pH-Wert von 6,6 herstellen. Die Aufbruchsspannung war kleiner als 450 Volt, bei 300 Volt Abscheidespannung wurde eine Schichtdicke von 20 µm erreicht, die während 20 Minuten bei 160°C zu einer harten Lackschicht eingebrannt wurde.

3.) Zu 2370 T der Verbindung III.1.) (2 Äquiv. Carbonat) wurden 204 T N,N-Dimethylaminopropylamin bei 60-80°C gegeben und die Reaktion bis zu einer Aminzahl von ungefähr 60 mg KOH/g Festharz fortgeführt. Das Reaktionsgut wurde mit Methoxypropanol auf einen Festkörpergehalt von 60 % eingestellt. MEQ-Wert 50 (15 %ige Klarlacklösung) pH-Wert von 7,3, Aufbruchsspannung ungefähr 250 Volt.

4.) Zu einer Mischung aus 7594 T der Verbindung V.1) (4 Äquiv. Carbonat, 70%ig), 1310 T eines Biscarbonats auf Basis von (R)Epikote 1001 (2 Äquiv. Carbonat, 80 %ig in Diglykoldimethylether) wurde bei 60 - 90°C eine Mischung,

bestehend aus 210 T Tetraethylenpentamin (2 Äquiv. prim. Amin), 176 T 4,7-Dioxadecan-1,10-diamin (2 Äquiv. prim. Amin), 131 T Dipropylentriamin (2 Äquiv. prim. Amin) und 14 T Methoxypropanol einlaufen gelassen und der Ansatz bis zu einer Aminzahl von ca. 33 mg KOH/g umgesetzt, Feststoffanteil 70 %.

5.) 860 T Bishexamethylentriamin (8 Äquiv. $NH_2$) wurden in 2315 T Methoxypropanol vorgelegt. Zu dieser Lösung wurden bei 20 - 40°C 622 T der Verbindung I.1) (2 Äquiv. NCO) einlaufen gelassen und bis zu einem NCO-Wert von ca. 0 % umgesetzt. Dann wurden 4737 T der Verbindung III.1) (4 Äquiv. Carbonat, 70%ig Diglykoldimethylether), 3246 T der Verbindung III.2) (2 Äquiv. Carbonat, 70 %ig Diglykoldimethylether) zugegeben und bei 60 - 90°C bis zu einer Aminzahl von ca. 32 mg KOH/g zur Reaktion gebracht, Feststoffanteil 60 %.

**VII. Prüfung der erfindungsgemäß hergestellten Bindemittel**

1.) Aus den Bindemitteln gemäß der Beispiele VI. 1 bis 5, wurden Klarlacke gemäß folgendem Ansatz hergestellt: Ansatz A: 286 T der entsprechenden Harzlösung X g 10 %ige wäßrige Ameisensäure entsprechend Tabelle 7, berechnet auf Festharz,

10 T Dibutylzinndilaurat

Einbrennbedingungen: 20 Minuten bei 160°C

Die Ansätze wurden jeweils mit deionisiertem Wasser auf einen Festkörpergehalt von ca. 15 % eingestellt. Die Abscheidedauer betrug jeweils 2 Minuten, die dafür benötigten Spannungen, die erzielte Filmdicke und die erzielten Eigenschaften sind in den Tabellen 5 und 5a angeführt.

2.) Aus dem Bindemittel entsprechend den Beispiel VI.5 wurde ein pigmentierter Lack gemäß folgendem Ansatz hergestellt:

Pigmentzusammensetzung

| | | |
|---|---|---|
| Ansatz B: | 84 T | Titandioxyd ((R)Kronos RN59 der Fa. Kronos-Titangesellschaft, Leverkusen) |
| | 1 T | Ruß (Raven 1170 der Firma Columbian Carbon, Hamburg) |
| | 10 T | Aluminiumsilicat ((R)Lanco ASP 200 der Firma Langer, Ritterhude) |
| | 5 T | Bleisilikat (EP 202 der Firma Chemag Frankfurt/Main) |
| | 100 T | |

Das angeführte Harz wurde mit Methoxypropanol auf einen Festkörpergehalt von 60 % eingestellt und mit der angeführten Pigmentmischung gemäß Ansatz B auf einem Dreiwalzenstuhl bis zu einer Korngröße von < 5 µm angerieben. Das Beispiel VI.5) wurde zur Bindemittelmischung gemäß Zusammensetzung C eingestellt.

| | | |
|---|---|---|
| C) | 750 | T Bindemittel gemäß Tabelle 6 |
| | 180 | T Pigmentmischung gemäß Schema B |
| | 22,5 | T Dibutylzinndilaurat |
| | X | T 10%ige Ameisensäure gemäß Tabelle 6 |
| | 9 | T eines Antikrater-Additivs ((R)Additol VXW 4922/280 der Firma Hoechst AG) |
| | 9 | T Verlaufmittel (Additol VXL 1359 der Fa. Hoechst AG) |
| | 18 | T 2,2,4-Trimethylpentandiol-1,3-monoisobutyrat |

Tabelle 5

| eingesetztes Harz VI. | 1 | 2 | 3 |
|---|---|---|---|
| Ameisensäure, 10%ig (T) | 46 | 46 | 46 |
| Bad-pH | 6,0 | 6,0 | 7,3 |
| max.Aufbruchspannung (V) | 300 | 400 | 250 |
| Abscheidespannung (V) [1] | 200 | 300 | 150-200 |
| Filmdicke (µm) | 10-15 | 12-14 | 10-15 |
| Verlauf [2] | 4-5 | 3-4 | 4-5 |
| Haftung [2] | 4-5 | 2-3 | 4-5 |

[1] bis 28-30°C

[2] 0 bester Wert

5 schlechtester Wert

Tabelle 5 (fortgesetzt)

| eingesetztes Harz VI. | 1 | 2 | 3 |
|---|---|---|---|
| Vernetzung 3) | 40-80 | 80-100 | 20-60 |
| Schlagtiefung 4) | 60 | 60 | 20 |

3) MEK-Doppelhülle, 1 kg Auflage
4) inch-pound

Tabelle 5a

| eingesetztes Harz VI. | 4 | 5 |
|---|---|---|
| Ameisensäure, 10%ig (T) | 32,2 | 46 |
| Bad-pH | 4,5 | 5,5 |
| max.Aufbruchspannung (V) | 200-250 | 400 |
| Abscheidespannung (V) 1) | 200 | 200 |
| Filmdicke (µm) | 25 | 20 |
| Verlauf 2) | 3 | 0 |
| Haftung 2) | 2 | 0 |
| Vernetzung 3) | 100 | 100 |
| Schlagtiefung 4) | 140 | 160 |

1) - 4) s. Tabelle 7

Tabelle 6

| eingesetztes Harz | Pigmentierungshöhe 1 : 0,4 |
|---|---|
| Beispiel | VI.5 |
| Ameisensäure, 10%ig (T) | 46 |
| Bad-pH | 5,7 |
| max. Aufbruchspannung (V) | 400 |
| Abscheidespannung (V) 1) | 300 |
| Filmdicke (µm) | 22 |
| Verlauf 2) | 0 |
| Haftung 2) | 0 |
| Vernetzung 3) | > 100 |
| Schlagtiefung 4) | 80-120 |
| Erichsen-Tiefung (mm) | 8-9 |

1)-4) s. Tabelle 7

**Patentansprüche**

1. Elektrotauchlacke auf Wasserbasis, enthaltend Aminourethane, die erhalten werden durch Umsetzung von oligomeren oder polymeren Verbindungen, die mindestens zwei endständige 2-Oxo-1,3-dioxolan-Gruppen enthalten, mit Aminen der Formel (I)

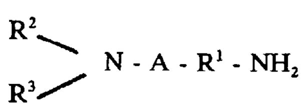

(I)

und/oder Aminen der Formel (Ia)

$$H_2N\text{-}A\text{-}R^1\text{-}NH_2 \qquad \text{(Ia)}$$

deren primäre Aminogruppe mit einem teilverkappten Polyisocyanat zu monoprimären Polyaminen umgesetzt wurde, wobei in den Formeln 1 und Ia

$R^1$ ein zweiwertiger Kohlenwasserstoffrest, vorzugsweise ein geradkettiger oder verzweigter Alkylenrest mit 2 bis 18 C-Atomen,

$R^2$ Wasserstoff, Alkyl mit 1 bis 8 C-Atomen oder Hydroxyalkyl mit 1 bis 8 C-Atomen im Alkylrest,

$R^3$ Alkyl mit 1 bis 8 C-Atomen oder Hydroxyalkyl mit 1 bis 8 C-Atomen im Alkylrest, wobei $R^2$ und $R^3$ auch eine cyclische Ringverbindung bilden können und

A eine chemische Bindung oder $-(R^1-NH)_r-NH-$ ist, worin r Null oder eine ganze Zahl von 1 bis 6 ist und $R^1$ die obige Bedeutung hat, und wobei zumindest ein Teil der basischen Aminogruppen dieser Aminourethane neutralisiert ist.

**2.** Elektrotauchlacke nach Anspruch 1, dadurch gekennzeichnet, daß die Menge an Verbindungen der Formeln I, bzw. Ia im Aminourethan 35 bis 85 Mol.-% und die Menge der Verbindungen, die mindestens zwei 2-Oxo-1,3-dioxolan-Gruppen enthalten, 65 bis 15 Mol.-% beträgt.

**3.** Elektrotauchlacke nach Anspruch 1 und/oder 2, dadurch gekennzeichnet, daß die Aminourethane die allgemeine Formel (II) besitzen

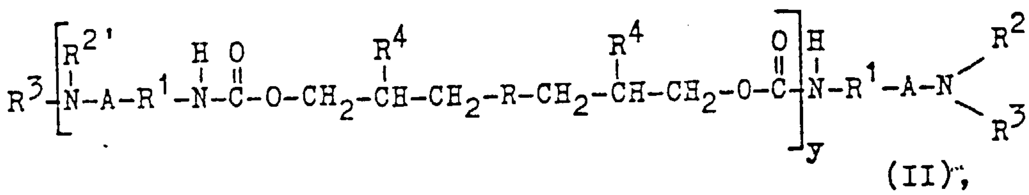

(II);

in der $R^1$, $R^2$ und $R^3$ sowie A die in Anspruch 1 genannte Bedeutung haben,

$R^{2'}$ gleich $R^2$ ist, mit der Maßgabe, daß $R^{2'}$ nur Wasserstoff darstellt, falls das betreffende Stickstoffatom nicht am Kettenende steht,

$R^4$ Hydroxyl oder den Rest

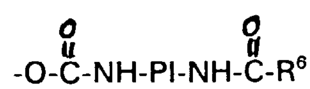

darstellt, in dem PI der Rest eines Polyisocyanats und $R^6$ der Rest eines aliphatischen, cycloaliphatischen oder alkylaromatischen einwertigen Alkohols, eines Aminoalkohols, eines Ketoxims, einer CH- oder NH-aciden Verbindung ist,

y eine ganze Zahl von 1 bis 10, vorzugsweise 1 bis 5 bedeutet und

R gleich und verschieden sein kann und für den Rest eines Diglycidylethers oder -esters, der gegebenenfalls auch $(NR_2)$-Gruppen enthalten kann, wobei $R^2$ die obige Bedeutung hat, oder für einen zweiwertigen Kohlenwasserstoffrest mit 2 bis 18 C-Atomen, steht.

**4.** Elektrotauchlacke nach einem oder mehreren der Ansprüche 1 bis 3, dadurch gekennzeichnet, daß die Aminourethane die allgemeine Formel (IIa) besitzen,

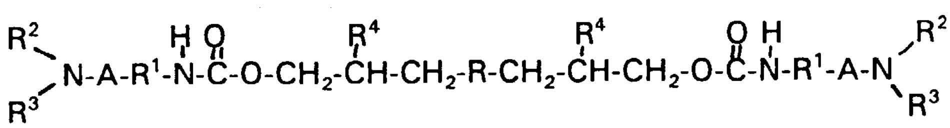

(IIa)

die aus der Formel II mit y = 1 hervorgeht.

**5.** Elektrotauchlacke nach Anspruch 3 und/oder 4, dadurch gekennzeichnet, daß R in den Formeln (II) bzw. (IIa) der Rest

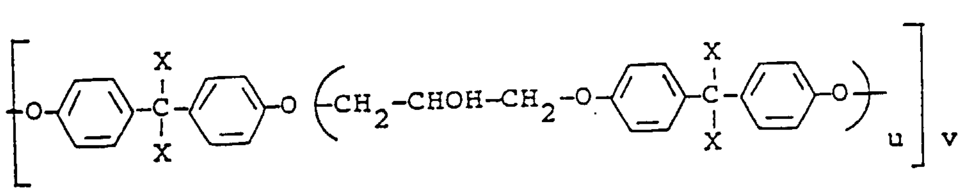

ist, in dem X Wasserstoff oder Methyl, u 0 bis 5 und v 1 bis 20 ist.

6. Elektrotauchlacke nach Anspruch 3 und/oder 4, dadurch gekennzeichnet, daß R in den Formeln (II) bzw. (IIa) den Rest

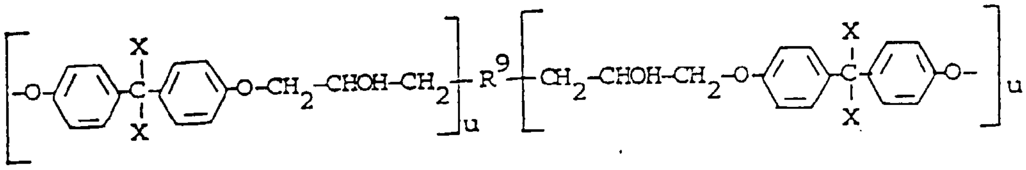

darstellt, worin X und u die genannte Bedeutung haben und $R^9$ O-Alkyl-O,

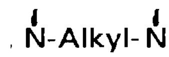

mit jeweils 2 bis 18 C-Atomen im Alkylrest oder der Rest von Polyaminen, Polyolen, Polycaprolactonpolyolen, OH-gruppenhaltigen Polyestern, Polyethern, hydroxy-, carboxyl- und aminofunktionellen Polymerölen, Polycarbonsäuren, hydroxy- oder aminofunktionellen Polytetrahydrofuranen und Reaktionsprodukten von Polyaminen mit Versaticsäureglycidylestern ist.

7. Elektrotauchlacke nach Anspruch 6, dadurch gekennzeichnet, daß $R^9$ den Rest

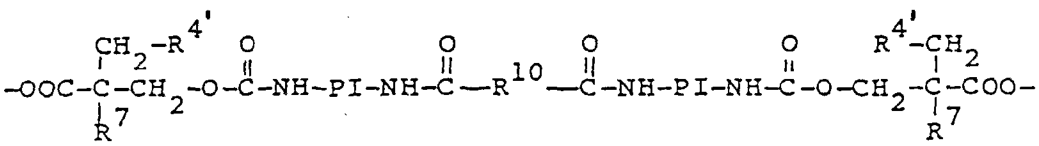

darstellt, wobei die Reste $R^7$ und PI die obige Bedeutung haben, $R^{4'}$ gleich $R^4$ ist und zusätzlich auch Wasserstoff sein kann, $R^{10}$ für die unter $R^9$ angeführten Reste mit Ausnahme der Polycarbonsäuren und der carboxylfunktionellen Polymeröle steht oder den Rest

$$-OOC-R^{11}-CO-R^{10}-CO-R^{11}-COO-$$

darstellt, in der $R^{10}$ wie vorstehend definiert ist und $R^{11}$ den aliphatischen, cycloaliphatischen oder aromatischen Rest einer Polycarbonsäure bedeutet.

8. Elektrotauchlacke nach Anspruch 3 und/oder 4, dadurch gekennzeichnet, daß R in den Formeln (II) bzw. (IIa) den Rest

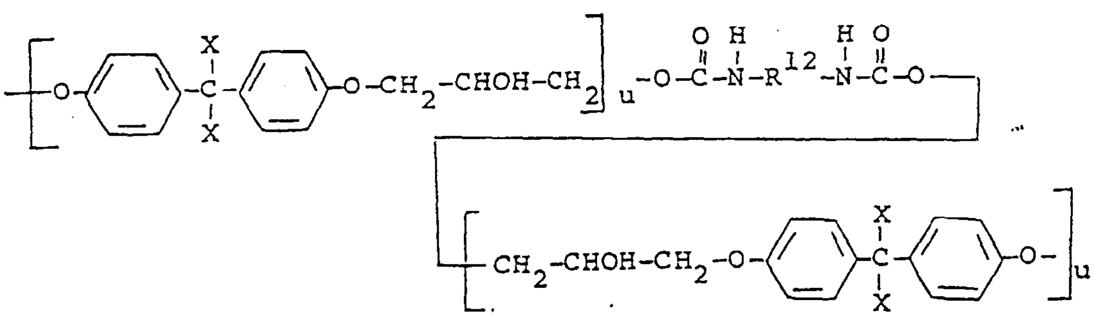

darstellt, worin X und u die genannte Bedeutung haben und $R^{12}$Alkylen mit 2 bis 18 C-Atomen, der Rest eines Poly(sek.)Amins oder aminofunktionellen Polytetrahydrofurans ist.

9. Elektrotauchlacke nach Anspruch 3 und/oder 4, dadurch gekennzeichnet, daß R in Formel (II) bzw. (IIa) den Rest

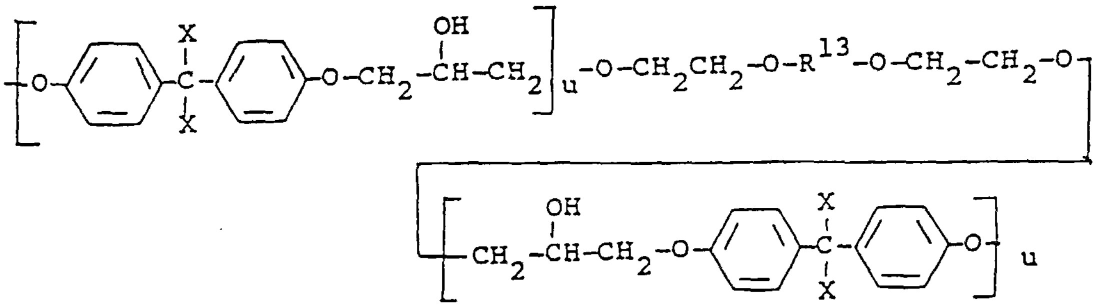

darstellt, worin X und u die genannte Bedeutung haben, u vorzugsweise aber 1 ist, und $R^{13}$ den Rest

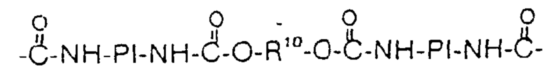

oder

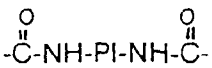

darstellt, wobei für $R^{10}$ und PI die schon genannten Definitionen gelten.

**10.** Elektrotauchlacke nach einem oder mehreren der Ansprüche 1 bis 9, dadurch gekennzeichnet, daß ein Teil oder alle der vorhandenen Hydroxyl- oder Aminogruppen mit teilverkappten Polyisocyanaten umgesetzt sind.

**11.** Elektrotauchlacke nach einem oder mehreren der Ansprüche 1 bis 10, dadurch gekennzeichnet, daß die oligomere oder polymere Verbindung, die mindestens zwei 2-Oxo-1,3-dioxolan-Gruppen enthält, die allgemeine Formel (III)

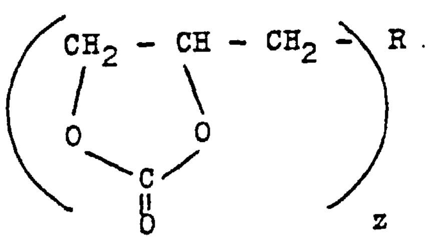

(III),

aufweist, in der R die Bedeutung gemäß Anspruch 1 besitzt, jedoch in seiner Wertigkeit z entspricht, und z für 2 bis 5 steht.

**12.** Elektrotauchlacke nach Anspruch 11, dadurch gekennzeichnet, daß die oligomere oder polymere Verbindung, die mindestens zwei 2-Oxo-1,3-dioxolan-Gruppen enthält, aus Epoxid-Carbonaten der allgemeinen Formel (IV)

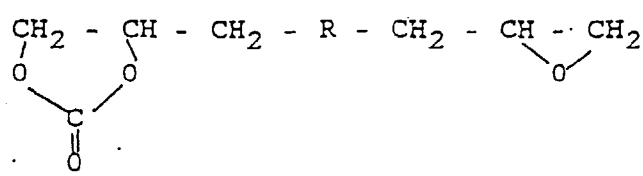

durch Umsatz von an die Epoxidgruppe addierbaren, multifunktionellen verbindungen erhalten wurde, wobei R die Bedeutung gemäß Anspruch 1 hat, und die Umsetzungen unter Bedingungen vorgenommen wurden, bei denen nur die Epoxidgruppen reagieren und die Carbonatgruppen nicht angegriffen wurden.

**13.** Substrate, beschichtet mit den Elektrotauchlacken gemäß einem oder mehreren der Ansprüche 1 bis 12.

## Claims

**1.** Water-based electrodeposition paints which contain aminourethanes obtained by reacting oligomeric or polymeric compounds containing at least two terminal 2-oxo-1,3-dioxolane groups with amines of the formula (I)

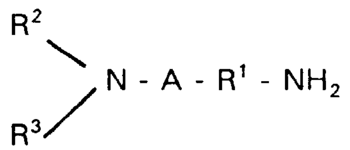 (I)

and/or with amines of the formula (Ia)

$$H_2N\text{-}A\text{-}R^1\text{-}NH_2 \qquad \text{(Ia)}$$

whose primary amino groups have been reacted with a partially blocked polyisocyanate to monoprimary polyamines, whereby, in formulae (I) and (Ia),

- $R^1$ denotes a divalent hydrocarbon radical, preferably a straight-chain or branched alkylene radical of 2 to 18 carbon atoms,
- $R^2$ denotes hydrogen, alkyl of 1 to 8 carbon atoms or hydroxyalkyl of 1 to 8 carbon atoms in the alkyl radical,
- $R^3$ denotes alkyl of 1. to 8 carbon atoms or hydroxyalkyl of 1 to 8 carbon atoms in the alkyl radical, it being possible for $R^2$ and $R^3$ to form a cyclic ring compound, and
- A denotes a chemical bond or $-(R^1\text{-}NH)_r\text{-}NH\text{-}$, with r denoting zero or an integer from 1 to 6 and $R^1$ having the meaning given above, and whereby at least part of the basic amino groups of said aminourethanes have been neutralized.

**2.** Electrodeposition paints as claimed in claim 1, wherein the amount of compounds of formulae (I) and/or (Ia) in the aminourethane is 35 to 85 mol-% and the amount of compounds containing at least two 2-oxo-1,3-dioxolane groups is 65 to 15 mol-%.

**3.** Electrodeposition paints as claimed in claims 1 and/or 2, wherein the aminourethanes have the general formula (II)

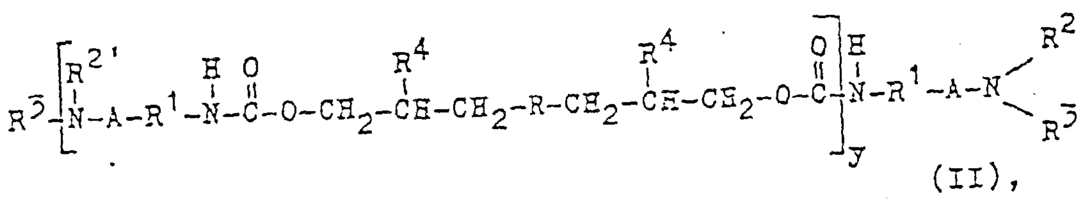 (II),

in which

| | |
|---|---|
| $R^1$, $R^2$ and $R^3$ | and A have the same meaning as in claim 1, |
| $R^{2'}$ | is identical to $R^2$ with the proviso that $R^{2'}$ only represents hydrogen if the respective nitrogen atom is not at the end of a chain, |
| $R^4$ | denotes hydroxyl or the radical 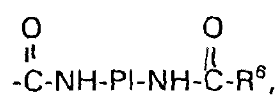 where PI is the radical of a polyisocyanate and $R^6$ is the radical of an aliphatic, cycloaliphatic or alkylaromatic monohydric alcohol, of an aminoalcohol, of a ketoxime, of a CH-acidic or of a NH-acidic compound, |
| y | denotes an integer from 1 to 10, preferably from 1 to 5, and |
| R | can be identical or different and stands for the radical of a diglycidyl ether or ester which may also contain $(NR^2)$-groups, where $R^2$ has the above meaning, or of a divalent hydrocarbon radical of 2 to 18 carbon atoms. |

**4.** Electrodeposition paints as claimed in one or several of claims 1 to 3, wherein the aminourethanes have the formula (IIa)

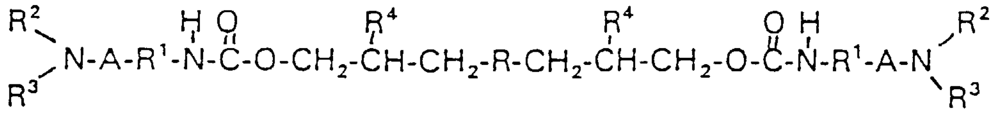 (IIa)

which is deducted from formula (II) with y = 1.

5. Electrodeposition paints as claimed in claims 3 and/or 4, wherein R in the formulae (II) and (IIa) is the radical

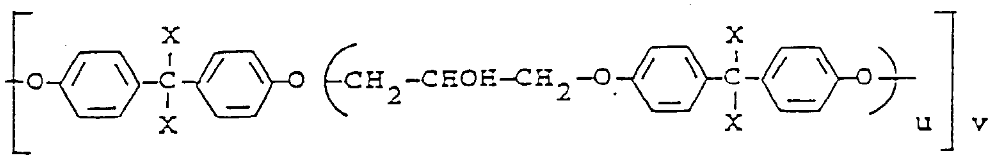

in which X is hydrogen or methyl, u is zero to 5 and v is 1 to 20.

6. Electrodeposition paints as claimed in claims 3 and/or 4, wherein R in the formulae (II) and (IIa) represents the radical

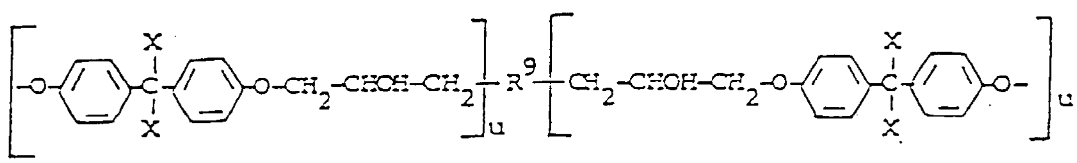

in which X and u have the meaning mentioned above and $R^9$ is O-alkyl-O,

N-alkyl-N

having in each case 2 to 18 carbon atoms in the alkyl radical or the radical of polyamines, polyols, polycaprolactonepolyols, OH-containing polyesters, polyethers, hydroxyl-, carboxyl- and amino-functional polymer oils, polycarboxylic acids, hydroxyl- or amino-functional polytetrahydrofurans and reaction products of polyamines with glycidyl esters of Versatic acid.

7. Electrodeposition paints as claimed in claim 6, wherein $R^9$ represents the radical

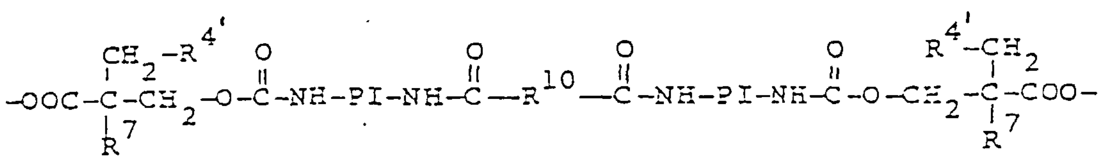

where the radicals $R^7$ and PI have the above meaning, $R^4$ is equal to $R^4$ and can additionally be hydrogen, $R^{10}$ stands for the radicals listed under $R^9$ with the exception of the polycarboxylic acids and the carboxylfunctional polymer oils or represents the radical

$$-OOC-R^{11}-CO-R^{10}-CO-R^{11}-COO-$$

in which $R^{10}$ is as defined above and $R^{11}$ denotes the aliphatic, cycloaliphatic or aromatic radical of a polycarboxylic acid.

8. Electrodeposition paints as claimed in claims 3 and/or 4, wherein R in the formulae (II) and (IIa) is the radical

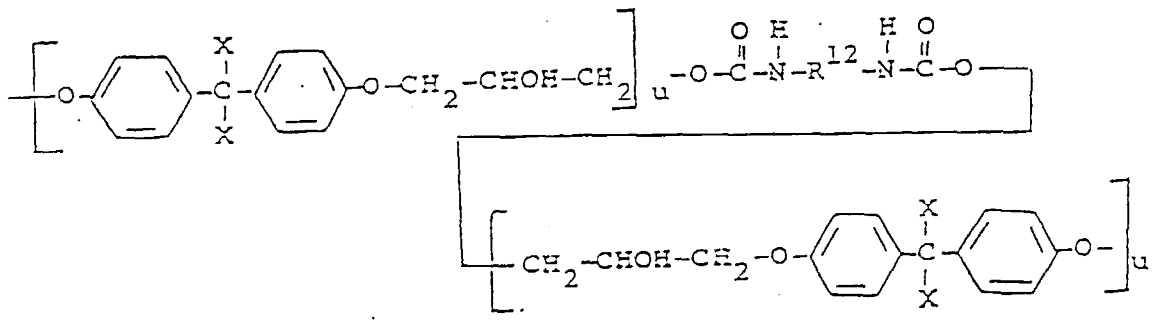

in which X and u have the meaning mentioned above and $R^{12}$ is alkylene of 2 to 18 carbon atoms or the radical of a poly(sec.)amine or of an amino-functional polytetrahydrofuran.

**9.** Electrodeposition paints as claimed in claims 3 and/or 4, wherein R in the formula (II) or (IIa) is the radical

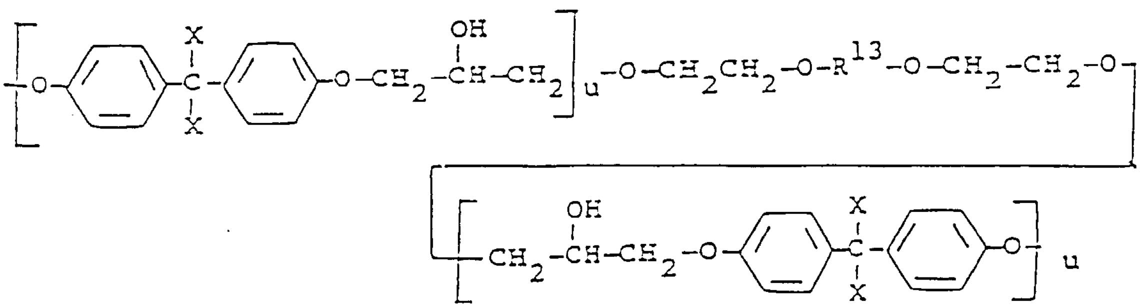

in which X and u have the meaning mentioned above, but u is preferably 1, and $R^{13}$ represents the radical

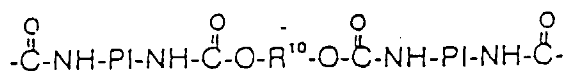

oder

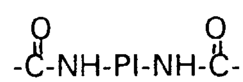

where $R^{10}$ and PI have the previously mentioned definitions.

**10.** Electrodeposition paints as claimed in one or more of claims 1 to 9, wherein some or all of the hydroxyl or amino groups present have been reacted with partially blocked polyisocyanates.

**11.** Electrodeposition paints as claimed in one or more of claims 1 to 10, wherein the oligomeric or polymeric compound containing at least one 2-oxo-1,3-dioxolane group has the formula (III)

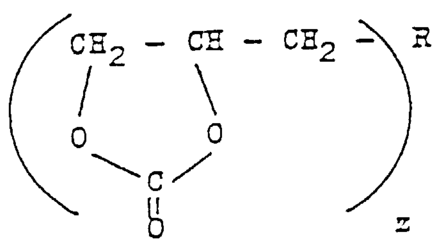

(III),

in which R has the meaning claimed in claim 1 but corresponds in its valence to z, and z stands for 2 to 5.

**12.** Electrodeposition paints as claimed in claim 11, wherein the oligomeric or polymeric compound containing at least two 2-oxo-1,3-dioxolane units has been prepared from epoxy carbonates of the formula (IV)

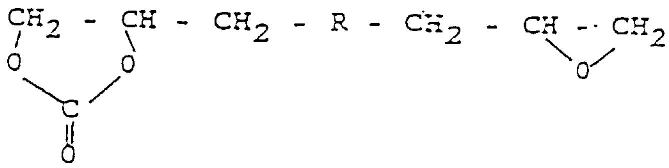 (IV)

by conversion of multifunctional compounds which are addable onto the epoxy group, R corresponding to the meaning given in claim 1 and the conversions being carried out under conditions where only the epoxy groups react and the carbonate groups are not attacked.

**13.** Substrates coated with electrodeposition paints as claimed in one or more of claims 1 to 12.

**Revendications**

**1.** Vernis de trempe électrophorétiques à base d'eau contenant des amino-uréthannes, que l'on obtient par réaction de composés oligomères ou polymères qui ont au moins deux groupes terminaux 2-oxo-1,3-dioxolannes, avec des amines de formule (I)

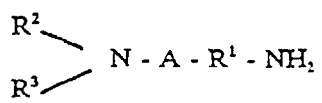 (I)

et/ou des amines de formule (Ia)

$$H_2N\text{-}A\text{-}R^1\text{-}NH_2 \quad \text{(Ia)}$$

dont ont fait réagir les groupes amino primaires avec un polyisocyanate partiellement bloqué pour obtenir des polyamines monoprimaires, dans les formules (I) et (Ia) étant :

- $R^1$ un radical hydrocarboné bivalent, de préférence un radical alkylène linéaire ou ramifié, avec 2 à 18 atoms de carbone,
- $R^2$ un atome d'hydrogène, alkyle avec 1 à 8 atomes de carbone ou hydroxyalkyle avec 1 à 8 atomes de carbone dans le radical alkyle
- $R^3$ alkyle avec 1 à 8 atomes de carbone ou hydroxyalkyle avec 1 à 8 atomes de carbone dans le radical alkyle, $R^2$ et $R^3$ pouvant former également un composé cyclique et
- A une liaison chimique ou $-(R^1\text{-}NH)_r\text{-}NH\text{-}$, où r vaut zéro ou est un nombre entier de 1 à 6 et $R^1$ a la signification donnée ci-dessus, et au moins une partie des groupes amino basiques de cet amino-uréthanne étant neutralisés.

**2.** Vernis de trempe électrophorétiques selon la revendication 1, caractérisés en ce que la quantité de composés de formules (I), respectivement (Ia) dans l'amino-uréthanne est de 35 à 85 % en moles et la quantité de composés qui contiennent au moins deux groupes 2-oxo-1,3-dioxolannes est de 65 à 15 % en moles.

**3.** Vernis de trempe électrophorétiques selon la revendication 1 et/ou 2, caractérisés en ce que les amino-uréthannes répondent à la formule générale (II)

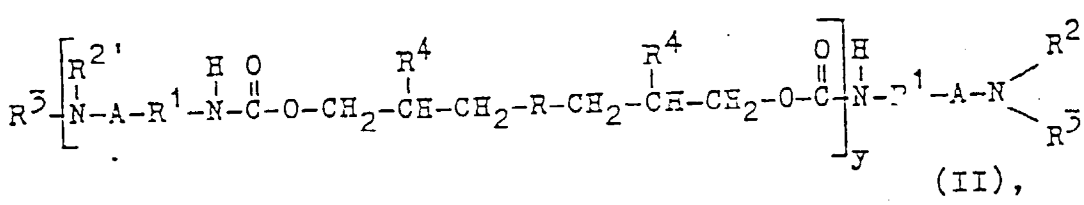 (II),

dans laquelle $R^1$, $R^2$ et $R^3$ ainsi que A ont les définitions données dans la revendication 1, $R^{2'}$ est égal à $R^2$, à la condition que $R^{2'}$ ne représente l'hydrogène que dans le cas où les atomes d'azote concernés ne se trouvent pas à l'extrémité de la chaîne,

$R^4$ représente hydroxyle ou le radical

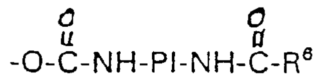

dans lequel PI représente le radical d'un polyisocyanate et $R^6$ le radical d'un alcool monovalent aliphatique, cycloaliphatique ou alkylaromatique, d'un amino-alcool, d'un cetoxime, d'un composé d'acide CH- ou NH-, y est un nombre entier de 1 à 10, de préférence de 1 à 5, et les radicaux
R peuvent être identiques ou différents et peuvent être un radical d'un éther ou d'un ester diglycidylique, qui peut éventuellement contenir des groupes $(NR^2)$-, $R^2$ ayant la signification donnée ci-dessus, ou étant un radical hydrocarboné bivalent avec 2 à 18 atomes de carbone,

**4.** Vernis de trempe électrophorétiques selon l'une des revendications 1 à 3, caractérisés en ce que les amino-uréthannes répondent à la formule générale (IIa)

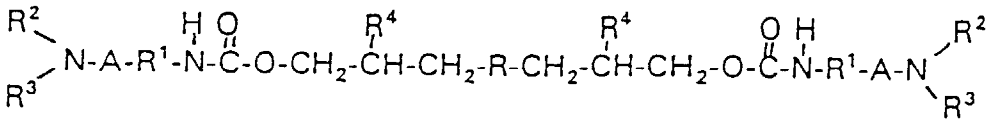

(IIa)

qui est déduite de la formule II avec y = 1.

**5.** Vernis de trempe électrophorétiques selon la revendication 3 et/ou 4, caractérisés en ce que R dans les formules (II), respectivement (IIa) est le radical

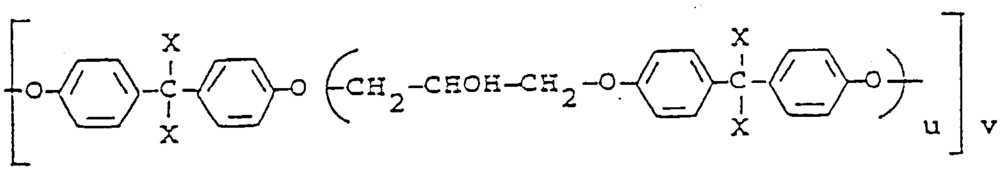

dans lequel X est l'hydrogène ou méthyle, u va de 0 à 5 et v de 1 à 20.

**6.** Vernis de trempe électrophorétiques selon la revendication 3 et/ou 4, caractérisés en ce que R dans les formules (II), respectivement (IIa) représente le radical

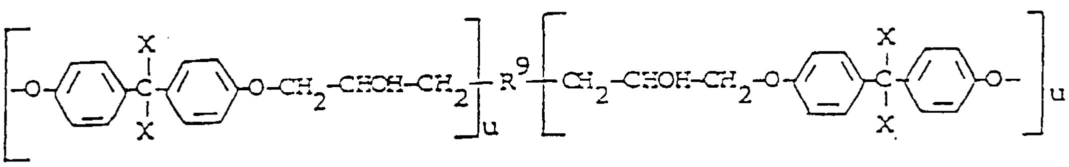

où X et u ont la signification donnée et $R^9$ représente O-alkyl-O,

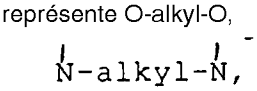

, avec chaque fois 2 à 18 atomes de carbone dans le radical alkyle ou représente les radicaux polyamines, polyols, polycaprolactonepolyols, polyesters contenant des groupes OH, polyéthers, huiles polymères à fonctionnalité hydroxyle, carboxyle ou amino, acides polycarboxyliques, polytétrahydrofurannes à fonctionnalité hydroxyle ou amino et des produits de réaction de polyamines avec des esters glycidyliques d'acides versatiques.

**7.** Vernis de trempe électrophorétiques selon la revendication 6, caractérisés en ce que $R^9$ représente le radical

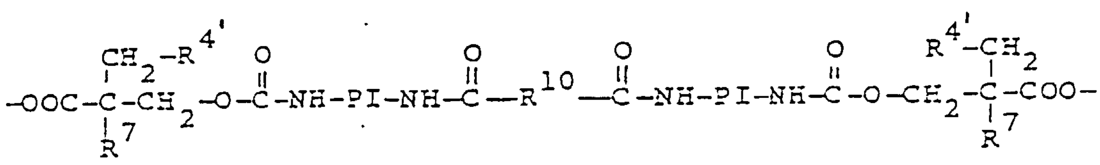

où les radicaux $R^7$ et PI ont la signification donnée ci-desssus, $R^{4'}$ est égal à $R^4$ et de plus, peut aussi être l'hydrogène, $R^{10}$ représente les radicaux indiqués sous $R^9$ à l'exception des acides polycarboxyliques et les huiles 5 polymères à fonctionnalité polycarboxyle ou le radical

$$-OOC-R^{11}-CO-R^{10}-CO-R^{11}-COO-$$

dans laquelle $R^{10}$ est définie comme ci-dessus et $R^{11}$ représente le radical aliphatique, cycloaliphatique ou aromatique d'un acide polycarboxylique.

**8.** Vernis de trempe électrophorétiques selon la revendication 3 et/ou 4, caractérisés en ce que R dans les formules (II), respectivement (IIa) représente le radical

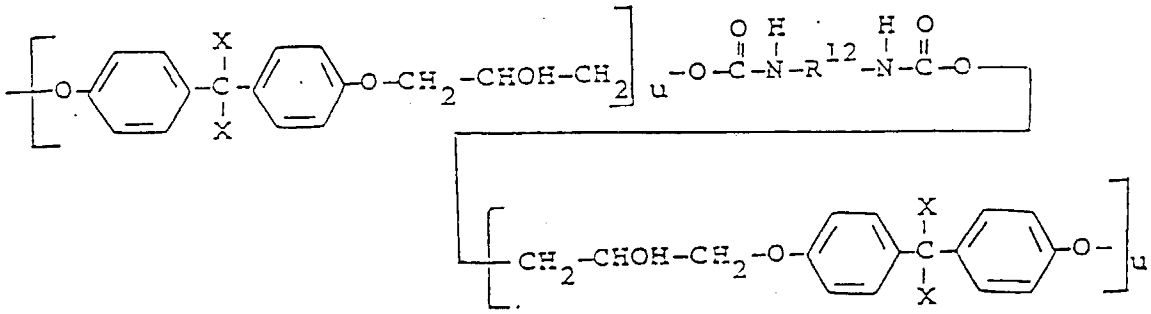

où X et u ont les signifcations données et $R^{12}$ est un alkylène avec 2 à 18 atomes de carbone, le radical d'une polo(sec)amine ou d'un polytétrahydrofuranne à fonctionnalité amino.

**9.** Vernis de trempe électrophorétiques selon la revendication 3 et/ou 4, caractérisé en ce R dans les formules (II), respectivement (IIa) représente le radical

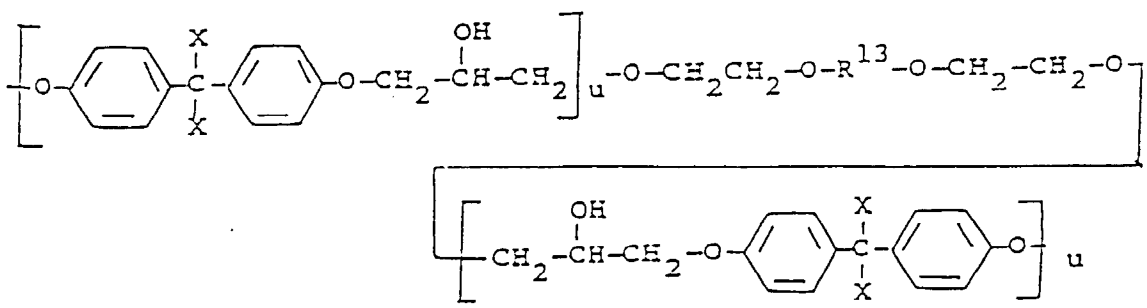

dans laquelle X et u ont les significations données, mais u étant de préférence 1, et $R^{13}$ représente le radical

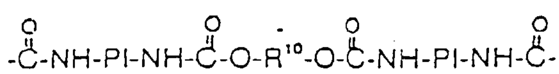

ou

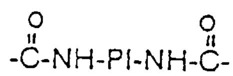

$R^{10}$ et PI étant définis selon les définitions déjà citées.

**10.** Vernis de trempe électrophorétiques selon une ou plusieurs des revendications 1 à 9, caractérisés en ce qu'on fait réagir une partie ou tous les groupes hydroxyles ou amino avec des polyisocyanates partiellement bloqués.

**11.** Vernis de trempe électrophorétiques selon une ou plusieurs des revendications 1 à 10, charactérisés en ce que le composé oligomère ou polymère qui présente au moins deux groupes 2-oxo-1, 3-dioxolanne a la formule générale (III)

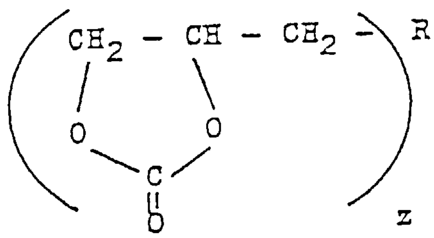 (III),

dans laquelle R a la signification selon la revendication 1, mais dont la valence correspond à z et s va de 2 à 5.

**12.** Vernis de trempe électrophorétiques selon la revendication 11, caractérisés en ce que l'on obtient le composé polymère ou oligomère, qui contient au moins deux groupes 2-oxo-1,3-dioxolannes à partir des époxy-carbonates de formule générale IV

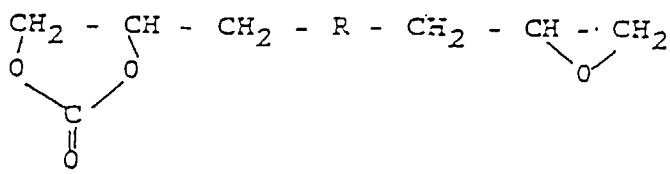

par réaction d'un composé multifonctionnel que l'on peut additionner sur le groupe époxyde, R ayant la signification selon la revendication 1 et la réaction étant mise en oeuvre dans des conditions où ne réagissent que les groupes époxydes et les groupes carbonate ne sont pas touchés.

**13.** Substrats enduits de vernis de trempe électro phorétiques selon une ou plusieurs des revendications 1 à 12.